# EUROPEAN PATENT APPLICATION

(11) **EP 4 825 328 A1**
(43) Date of publication of application: **21.10.2026**
(21) Application number: 24903301.0
(22) Date of filing: 08.10.2024
(51) Int. Cl.: A61B 17/86, A61B 17/70, F16B 25/00

(54) **BONE SCREW AND BONE SCREW MANUFACTURING METHOD**

(30) Priority: 15.12.2023 JP 2023211736
(71) Applicant: Spine-Tech Inc., Tokyo 105-0003 (JP); Takai Corporation, Mino-Shi, Gifu 501-3712 (JP)
(72) Inventor: MORITA, Kohei, Tokyo 105-8461 (JP); TSUCHIDA, Kenji, Mino-shi, Gifu 501-3712 (JP); FURUTA, Yasuhiro, Mino-shi, Gifu 501-3712 (JP)
(74) Representative: SSM Sandmair
(86) International application number: PCT/JP2024/036002
(87) International publication number: WO 2025/126638

(57) **Abstract**

It is possible to provide a bone screw in which a sliding portion, a fixing portion, or a abutting surface between different materials or parts does not easily slide or break. The bone screw of the present invention comprises a shaft, a thread extending spirally along an axial direction of the shaft on an outer peripheral portion of the shaft, a first end located at an insertion-direction end of the shaft, a second end located at a removal-direction end of the shaft, a head positioned to surround the second end, and a fixing member attached to the head and configured to fix a rod member to the head, wherein at least one of the second end, a sliding portion of the head configured to slide against the second end, and the fixing member has an arithmetic surface roughness (Ra) of 0.4 µm to 1.7 µm.

## Description

### TECHNICAL FIELD

The present invention relates to a bone screw and a method for manufacturing a bone screw.

### BACKGROUND OF INVENTION

Bone screws are used in fracture surgeries and spinal surgeries. For example, the spine is stabilized by connecting pedicle screws inserted into pedicles with rods or the like.

### CITATION LIST

### PATENT LITERATURE

Patent Document 1: JP2019-76755A

### SUMMARY

### [Problem to be solved]

Bone screws used during surgery and during the period of use are required to have a certain degree of durability. For example, it is preferable that a bone screw inserted into bone does not deform even when a certain force is applied. It is preferable that the sliding portion or the fixing portion does not easily slide even when a certain force is applied during surgery or during the period of use. Further, with respect to bone screws composed of a plurality of materials or parts, it is preferable that joints between different materials or parts do not easily slide or break even when a certain force is applied during surgery or during the period of use.

A bone screw according to the present invention comprises a shaft, a thread extending spirally along the axial direction of the shaft on an outer peripheral portion of the shaft, a first end (distal end) located at an insertion-direction end of the shaft, a second end (proximal end) located at a removal-direction end of the shaft, a head positioned to surround the second end, and a fixing member attached to the head and capable of fixing a rod member to the head, wherein at least one of the second end, a sliding portion of the head slidable against the second end, and the fixing member has an arithmetic surface roughness (Ra) in a range of 0.4 µm to 1.7 µm.

The present invention provides a bone screw in which loosening or breakage of a sliding portion, a fixing portion, or an abutting surface between different materials or parts is prevented.

### BRIEF DESCRIPTION OF THE DRAWINGS

Figure 1 is a front view of the bone screw of the present embodiment.
Figure 2 is a cross-sectional view of the bone screw of the present embodiment.
Figure 3 is a perspective view of the bone screw of the present embodiment.
Figure 4A is a top view of the bone screw of the present embodiment. Figure 4B is a bottom view of the bone screw of the present embodiment.
Figure 5 is a view showing a flexion-extension moment test.
Figure 6 is a view showing results of a flexion-extension moment test of a bone screw of a comparative example and the bone screw of the present embodiment.
Figure 7 is a view showing a torsion test.
Figure 8 is a view showing results of a torsion test of a bone screw of a comparative example and the bone screw of the present embodiment.

### DESCRIPTION OF EMBODIMENTS

The inventors found a surface roughness at which, in a bone screw having a sliding portion, the sliding portion or fixing portion does not easily slide. The inventors also found a surface roughness at which, in a bone screw composed of a plurality of materials or parts, abutting surfaces between different materials or parts do not easily slide or break.

According to the present embodiment, it is possible to provide a bone screw in which a sliding portion, a fixing portion, or a abutting surface between different materials or parts does not easily slide or break.

With respect to the present embodiment, a medical bone screw fixed to a spine will be described with reference to the drawings. The bone screw of the present embodiment is used to treat spinal diseases, for example, spinal deformity, vertebral fracture, spinal canal stenosis, spinal dislocation, or spinal instability, in order to improve spinal stability. Further, the bone screw of the present embodiment is used to treat spinal diseases by inserting a plurality of bone screws into pedicles of the spine and connecting the plurality of bone screws with a rod member to properly support the spine or restrict movement of the spine.

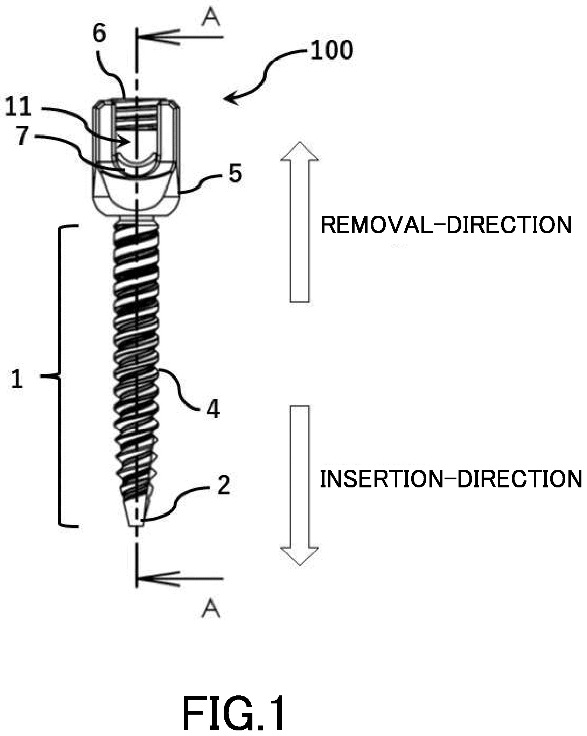

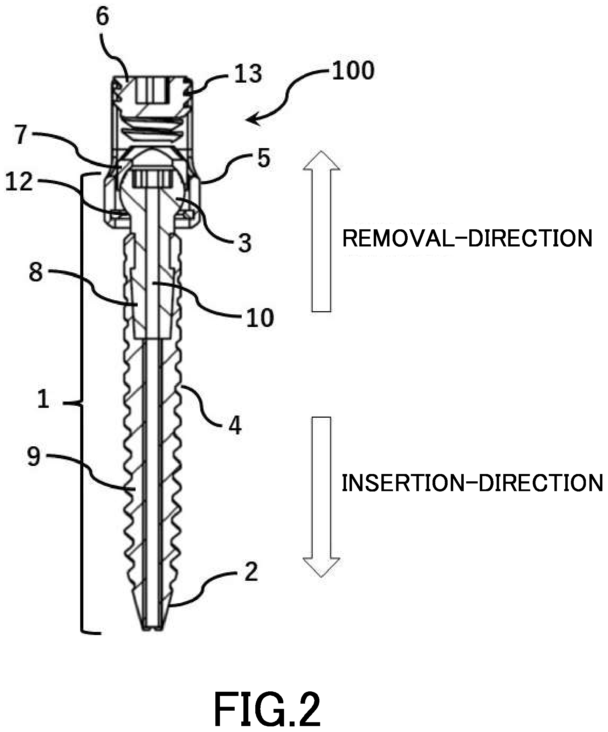

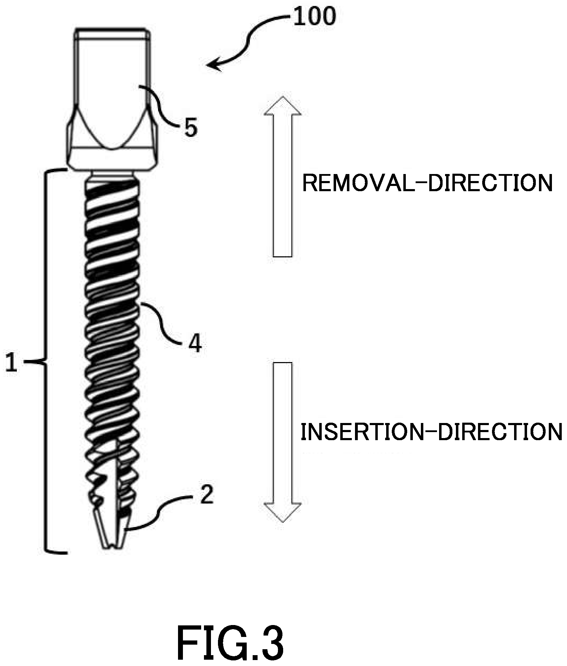

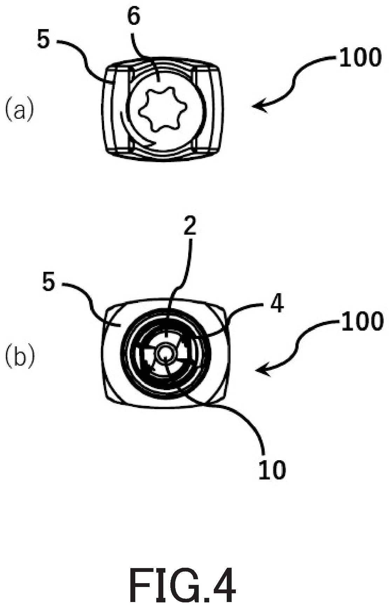

Fig. 1 is a front view of the bone screw of the present embodiment. Fig. 2 is a cross-sectional view taken along line A-A of Fig. 1. Fig. 3 is a perspective view of the bone screw of the present embodiment. Fig. 4A is a top view of the bone screw of the present embodiment. Fig. 4B is a bottom view of the bone screw of the present embodiment.

As shown in Figs. 1 to 4, the bone screw 100 includes a shaft 1 and a thread 4 extending spirally along the axial direction of the shaft 1 on an outer peripheral portion of the shaft 1. The bone screw 100 also includes a first end (distal end) 2 located at an insertion-direction end of the shaft 1 and a second end (proximal end) 3 located at a removal-direction end of the shaft 1. The bone screw 100 also includes a head 5 positioned to surround the second end. In the present embodiment, the head 5 includes a sliding portion configured to slide against the second end 3, but may be formed integrally or fixedly with the second end 3. The bone screw 100 also includes a fixing member (set screw) 6 serving as a fixing portion. The fixing member 6 is attached to the head 5 and is capable of fixing a rod member (not shown) to the head 5. The bone screw 100 also includes an abutting portion (pusher) 7 that abuts against the second end 3.

A rod member (not shown) connecting a plurality of bone screws is disposed in a recess 11 (Fig. 1) of the head 5 to contact the abutting portion 7, and the rod member is pressed and fixed by fastening the fixing member (male screw) 6 to a female screw 13 (Fig. 2) of the head 5. When the fixing member (male screw) 6 is fastened to the female screw 13 (Fig. 2) of the head 5, the abutting portion 7 presses down the second end 3 together with the rod member, whereby the second end 3 is pressed and fixed to the sliding portion 12 (Fig. 2) of the head 5.

At least one of the second end 3, the sliding portion 12 of the head 5 slidable against the second end 3, and the fixing member (male screw) 6 has an arithmetic surface roughness (Ra) in a range of 0.4 µm to 1.7 µm. For example, a portion of the second end 3 that contacts the sliding portion 12 (Fig. 2) of the head 5 has an arithmetic surface roughness (Ra) of 0.4 µm to 1.7 µm. Further, a portion of the sliding portion 12 (Fig. 2) of the head 5 that contacts the second end 3 has an arithmetic surface roughness (Ra) in a range of 0.4 µm to 1.7 µm. Further, a portion of the fixing member 6 that contacts the head 5 when the fixing member 6 is attached to the head 5 has an arithmetic surface roughness (Ra) in a range of 0.4 µm to 1.7 µm.

It should be noted that only one of the second end 3 and the sliding portion 12 of the head 5 slidable against the second end 3 may have an arithmetic surface roughness (Ra) in a range of 0.4 µm to 1.7 µm.

Further, the abutting portion 7 has an arithmetic surface roughness (Ra) in a range of 0.4 µm to 1.7 µm. For example, a portion of the abutting portion 7 that contacts the second end 3 has an arithmetic surface roughness (Ra) in a range of 0.4 µm to 1.7 µm.

Further, as shown in Fig. 2, the shaft 1 includes a shaft core portion 8 including the second end 3, and a shaft thread 9 located on an outer peripheral portion of the shaft core portion 8 and including the thread 4. Further, the shaft core portion 8 includes a through-hole 10 for allowing a guide wire or the like to pass therethrough. The arithmetic surface roughness (Ra) of the shaft core portion 8 contacting the shaft thread 9 is in a range of 0.4 µm to 1.7 µm.

The shaft core portion 8 (or the second end) includes at least one of stainless steel, titanium, iron, nickel, cobalt, and cemented carbide. The shaft thread 9 (or the thread 4) includes reinforced plastic. The reinforced plastic is at least one of carbon fiber reinforced plastic, glass fiber reinforced plastic, boron fiber reinforced plastic, aramid fiber reinforced plastic, Kevlar fiber reinforced plastic, Dyneema fiber reinforced plastic, and Zylon fiber reinforced plastic.

Next, a method for manufacturing the bone screw will be described. The bone screw manufacturing method of the present embodiment includes a step of performing surface treatment so that an arithmetic surface roughness (Ra) in a range of 0.4 µm to 1.7 µm is provided on at least one surface of the second end 3, the sliding portion 12 of the head slidable against the second end 3, and the fixing member 6. The bone screw manufacturing method of the present embodiment also includes a step of performing surface treatment so that an arithmetic surface roughness (Ra) in a range of 0.4 µm to 1.7 µm is provided on a surface of the shaft core portion 8 contacting the shaft thread 9. The bone screw manufacturing method of the present embodiment also includes a step of performing surface treatment so that an arithmetic surface roughness (Ra) in a range of 0.4 µm to 1.7 µm is provided on a surface of the abutting portion 7.

In the step of performing surface treatment, blast treatment is carried out. For example, blast treatment using white alundum, zircon beads, or the like is carried out. Further, separable parts may be subjected to blast treatment in a separated state. For example, when the shaft 1, the head 5, the fixing member 6, and the abutting portion 7 are separable, blast treatment may be carried out with each of them separated. Further, in the course of manufacturing the bone screw, blast treatment may be carried out on a part before different parts are attached. For example, the shaft core portion 8 may be subjected to blast treatment before the shaft thread 9 is attached. In this case, after the shaft core portion 8 is subjected to blast treatment, the shaft thread 9 may be injection-molded on the outer peripheral portion of the shaft core portion 8 with the shaft core portion 8 serving as an 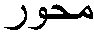 .

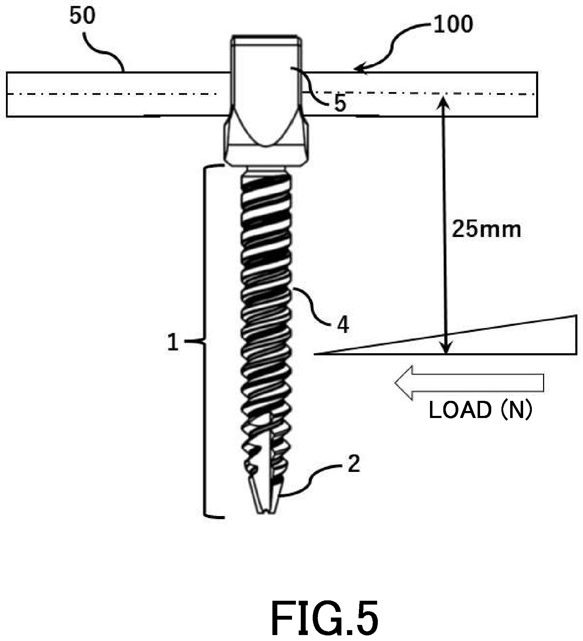

Next, the performance of the bone screw of the present embodiment will be described. As shown in Fig. 5, a flexion-extension moment test was carried out with reference to ASTM F1798. With the rod member 50 disposed in the recess 11 (Fig. 1) of the bone screw 100 and the fixing member 6 fastened to the head 5 with a torque of 8 Nm to 12 Nm, the rod member 50 was fixed, and a load (N) was applied at a position 25 mm from the central axis of the rod member 50. The test was terminated when the bone screw 100 was significantly deformed, the maximum load (N) up to the time of termination was measured, and the maximum load (N) was taken as bending strength. The second end 3 of the bone screw 100 of the present embodiment and the sliding portion of the head 5 slidable against the second end 3 (the sliding portion includes the abutting portion 7) mainly include titanium.

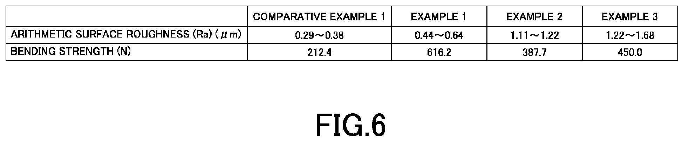

Fig. 6 is a view showing the results of the flexion-extension moment test of the bone screw of Comparative Example 1 and the bone screw of Examples 1-3 of the present embodiment. As shown in Fig. 6, in the case of the bone screw of Comparative Example 1, in which the arithmetic surface roughness (Ra) of the second end 3 and the sliding portion of the head 5 slidable against the second end 3 was in a range of 0.29 µm to 0.38 µm, the bending strength was 212.4 N.

In contrast, in Example 1 of the present embodiment, in which the arithmetic surface roughness (Ra) of the second end 3 and the sliding portion of the head 5 slidable against the second end 3 was 0.44 µm to 0.64 µm, the bending strength was 616.2 N. Further, in Example 2 of the present embodiment, in which the arithmetic surface roughness (Ra) of the second end 3 and the sliding portion of the head 5 slidable against the second end 3 was in a range of 1.11 µm to 1.22 µm, the bending strength was 387.7 N. Further, in Example 3 of the present embodiment, in which the arithmetic surface roughness (Ra) of the second end 3 and the sliding portion of the head 5 slidable against the second end 3 was in a range of 1.22 µm to 1.68 µm, the bending strength was 450.0 N.

As a result, when at least one of the second end 3 and the sliding portion 12 of the head slidable against the second end 3 had an arithmetic surface roughness (Ra) in a range of 0.4 µm to 1.7 µm, the bending strength was 380 N to 620 N, and sufficient bending strength could be obtained as compared with Comparative Example 1. Preferably, when at least one of the second end 3 and the sliding portion 12 of the head 5 slidable against the second end 3 had an arithmetic surface roughness (Ra) in a range of 1.2 µm to 1.7 µm, the bending strength was 450 N, and sufficient bending strength could be obtained as compared with Comparative Example 1. More preferably, when at least one of the second end 3 and the sliding portion 12 of the head 5 slidable against the second end 3 had an arithmetic surface roughness (Ra) in a range of 0.4 µm to 0.7 µm, the bending strength was 616.2 N, and sufficient bending strength could be obtained as compared with Comparative Example 1.

In Comparative Example 1, loosening of the fixing member 6 was observed, whereas in Examples 1 to 3 no loosening of the fixing member 6 was observed. As a result, when the arithmetic surface roughness (Ra) of the fixing member 6 was in a range of 0.4 µm to 1.7 µm, sufficient fastening strength (fixing strength) could be obtained as compared with Comparative Example 1. Preferably, the arithmetic surface roughness (Ra) of the fixing member 6 is in a range of 1.2 µm to 1.7 µm. More preferably, the arithmetic surface roughness (Ra) of the fixing member 6 is in a range of 0.4 µm to 0.7 µm.

Further, from the results of the above flexion-extension moment test, when the arithmetic surface roughness (Ra) of the abutting portion 7 that abuts against the second end 3, which corresponds to a part of the sliding portion of the head 5 slidable against the second end 3, was in a range of 0.4 µm to 1.7 µm, sufficient bending strength could be obtained as compared with Comparative Example 1. Preferably, the arithmetic surface roughness (Ra) of the abutting portion 7 that abuts against the second end 3 is 1.2 µm to 1.7 µm. More preferably, the arithmetic surface roughness (Ra) of the abutting portion 7 that abuts against the second end 3 is in a range of 0.4 µm to 0.7 µm.

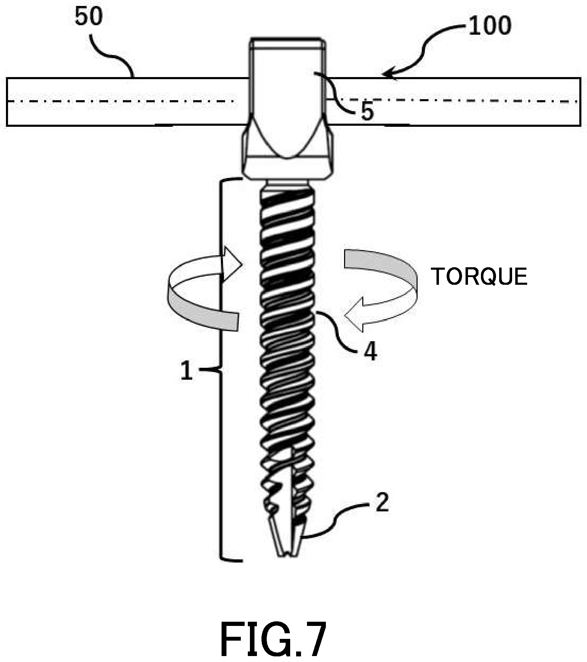

Next, as shown in Fig. 7, a torsion test was carried out. With the rod member 50 disposed in the recess 11 (Fig. 1) of the bone screw 100 and the fixing member 6 fastened to the head 5 with a torque of 8 Nm to 12 Nm, the rod member 50 was fixed, and torque (Nm) was applied to the shaft thread 9 (or the thread 4). The test was terminated when the bone screw 100 was significantly deformed, the maximum torque (Nm) up to the time of termination was measured, and the maximum torque (Nm) was taken as torsional strength. The shaft core portion 8 of the bone screw 100 of the present embodiment, or the portion of the shaft core portion 8 contacting the shaft thread 9, mainly includes titanium, and the shaft thread 9, or the portion of the shaft thread 9 contacting the shaft core portion 8, mainly includes carbon fiber reinforced plastic.

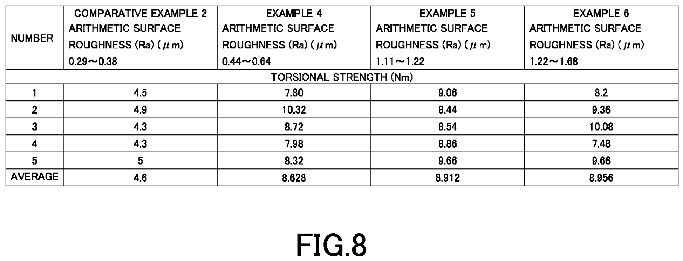

Fig. 8 is a view showing the results of the torsion test of the bone screw of Comparative Example 2 and the bone screw of Examples 4-6 of the present embodiment. As shown in Fig. 8, in the case of the bone screw of Comparative Example 2, in which the arithmetic surface roughness (Ra) of the shaft core portion 8 contacting the shaft thread 9 was in a range of 0.29 µm to 0.38 µm, the average torsional strength was 4.6 Nm.

In contrast, in Example 4 of the present embodiment, in which the arithmetic surface roughness (Ra) of the shaft core portion 8 contacting the shaft thread 9 was in a range of 0.44 µm to 0.64 µm, the average torsional strength was 8.628 Nm. Further, in Example 5 of the present embodiment, in which the arithmetic surface roughness (Ra) of the shaft core portion 8 contacting the shaft thread 9 was in a range of 1.11 µm to 1.22 µm, the average torsional strength was 8.912 Nm. Further, in Example 6 of the present embodiment, in which the arithmetic surface roughness (Ra) of the shaft core portion 8 contacting the shaft thread 9 was in a range of 1.22 µm to 1.68 µm, the average torsional strength was 8.956 Nm.

As a result, when the arithmetic surface roughness (Ra) of the shaft core portion 8 contacting the shaft thread 9 was in a range of 0.4 µm or more, sufficient torsional strength could be obtained as compared with Comparative Example 2. Preferably, the arithmetic surface roughness (Ra) of the shaft core portion 8 contacting the shaft thread 9 is in a range of 0.4 µm to 1.7 µm. More preferably, the arithmetic surface roughness (Ra) of the shaft core portion 8 contacting the shaft thread 9 is in a range of 1.1 µm to 1.7 µm. Still more preferably, the arithmetic surface roughness (Ra) of the shaft core portion 8 contacting the shaft thread 9 is in a range of 1.2 µm to 1.7 µm.

In consideration of both the flexion-extension moment test (Fig. 6) and the torsion test (Fig. 8) of the bone screw of the present embodiment, when the second end 3 and the shaft core portion 8 of the bone screw are formed integrally or fixedly, it is preferable that the arithmetic surface roughness (Ra) of the second end 3 and the shaft core portion 8 be matched within an appropriate range, because the second end 3 and the shaft core portion 8 can be surfaceprocessed simultaneously, thereby reducing man-hours and improving production efficiency.

Accordingly, it is preferable that the arithmetic surface roughness (Ra) of the second end 3 and the shaft core portion 8 of the bone screw be in a range of 0.4 µm to 0.7 µm or 1.2 µm to 1.7 µm. More preferably, the arithmetic surface roughness (Ra) of the second end 3 and the shaft core portion 8 of the bone screw is in a range of 0.4 µm to 0.7 µm.

This makes it possible to improve production efficiency by surface-processing the second end 3 and the shaft core portion 8 of the bone screw simultaneously, and also to obtain sufficient bending strength and torsional strength.

The embodiments according to the present invention have been described above; however, the present invention is not limited thereto, and modifications and variations may be made within the scope described in the claims.

For example, in the step of performing surface treatment, in addition to blast treatment, etching, laser treatment, mechanical processing such as milling, needling, and sanding, polishing treatment such as shot peening, thermal spraying, and electrochemical processing such as anodizing may also be used.

The present invention is useful as a bone screw in which a sliding portion, a fixing portion, or a abutting surface between different materials or parts does not easily slide or break.

### [Description of Reference Numerals]

1 shaft
2 first end
3 second end
4 thread
5 head
6 fixing member
7 abutting portion
8 shaft core portion
9 shaft thread
10 through-hole
11 recess
12 sliding portion
13 female screw
100 bone screw

## Claims

1. A bone screw comprising:
a shaft;
a thread extending spirally along an axial direction of the shaft on an outer peripheral portion of the shaft;
a first end located at an insertion-direction end of the shaft;
a second end located at a removal-direction end of the shaft;
a head positioned to surround the second end; and
a fixing member attached to the head and configured to fix a rod member to the head,
**characterized in that**
at least one of the second end, a sliding portion of the head configured to slide against the second end, and the fixing member has an arithmetic surface roughness (Ra) in a range of 0.4 µm to 1.7 µm.

2. A bone screw comprising:
a shaft;
a thread extending spirally along an axial direction of the shaft on an outer peripheral portion of the shaft;
a first end located at an insertion-direction end of the shaft;
a second end located at a removal-direction end of the shaft,
wherein the shaft includes:
a shaft core portion including the second end; and
a shaft thread located on an outer peripheral portion of the shaft core portion and including the thread,
**characterized in that**
the arithmetic surface roughness (Ra) of the shaft core portion contacting the shaft thread is in a range of 0.4 µm to 1.7 µm.

3. A bone screw comprising:
a shaft;
a thread extending spirally along an axial direction of the shaft on an outer peripheral portion of the shaft;
a first end located at an insertion-direction end of the shaft;
a second end located at a removal-direction end of the shaft; and
an abutting portion configured to abut against the second end,
**characterized in that**
the abutting portion has an arithmetic surface roughness (Ra) in a range of 0.4 µm to 1.7 µm.

4. The bone screw according to any one of Claims 1 to 3, **characterized in that** the thread includes reinforced plastic.

5. The bone screw according to Claim 4, **characterized in that** the reinforced plastic is at least one of carbon fiber reinforced plastic, glass fiber reinforced plastic, carbon-fiber reinforced plastic, boron fiber reinforced plastic, aramid fiber reinforced plastic, Kevlar fiber reinforced plastic, Dyneema fiber reinforced plastic, and Zylon fiber reinforced plastic.

6. The bone screw according to any one of Claims 1 to 3, **characterized in that** the second end includes at least one of stainless steel, titanium, iron, nickel, cobalt, and cemented carbide.

7. A method for manufacturing a bone screw comprising:
providing a shaft;
forming a thread extending spirally along an axial direction of the shaft on an outer peripheral portion of the shaft;
providing a first end located at an insertion-direction end of the shaft;
providing a second end located at a removal-direction end of the shaft;
providing a head positioned to surround the second end; and
providing a fixing member attached to the head and configured to fix a rod member to the head;
**characterized in that**
performing a surface treatment to provide an arithmetic surface roughness (Ra) in a range of 0.4 µm to 1.7 µm on at least one of:
a surface of the second end,
a sliding portion of the head configured to slide against the second end, and
the fixing member.

8. A method for manufacturing a bone screw, comprising:
providing a shaft having:
a shaft core portion including a second end located at a removal-direction end of the shaft; and
a shaft thread located on an outer peripheral portion of the shaft core portion, the shaft thread including a thread extending spirally along an axial direction of the shaft;
**characterized in that**
performing a surface treatment to provide an arithmetic surface roughness (Ra) in a range of 0.4 µm to 1.7 µm on a surface of the shaft core portion that contacts the shaft thread.

9. A method for manufacturing a bone screw, comprising:
providing a shaft;
forming a thread on an outer peripheral portion of the shaft, the thread extending spirally along an axial direction of the shaft;
providing a first end at an insertion-direction end of the shaft;
providing a second end at a removal-direction end of the shaft;
providing an abutting portion configured to abut against the second end; and
**characterized in that**:
performing a surface treatment to provide an arithmetic surface roughness (Ra) in a range of 0.4 µm to 1.7 µm on a surface of the abutting portion.
